# FASCICULE DE BREVET EUROPEEN

(11) **EP 1 148 904 B1**
(45) Date de publication et mention de la délivrance du brevet: **09.02.2005**
(21) Numéro de dépôt: 99923709.2
(22) Date de dépôt: 09.06.1999
(51) Int. Cl.: A61M 5/32

(54) **ENSEMBLE DE SECURITE POUR UNE SERINGUE PRE-REMPLIE D'INJECTION DE LIQUIDE, NOTAMMENT D'UN MEDICAMENT**
SICHERHEITSANORDNUNG FÜR SPRITZE VORGEFÜLLT MIT FLÜSSIGEM ARZNEIMITTEL
SAFETY ASSEMBLY FOR A SYRINGE PRE-FILLED WITH LIQUID, IN PARTICULAR A MEDICINE

(30) Priorité: 08.12.1998 FR 9815628
(43) Date de publication de la demande: 31.10.2001
(60) Demande divisionnaire: 03290416.1
(73) Titulaire: COMPAGNIE PLASTIC OMNIUM, 69007 Lyon (FR)
(72) Inventeur: DENIS, Roger, Décédé (FR)
(74) Mandataire: Remy, Vincent Noel Paul
(86) Numéro de dépôt international: PCT/FR1999/001369
(87) Numéro de publication internationale: WO 2000/033900

(56) Documents cités:
- EP-A- 0 405 039
- US-A- 4 932 947
- US-A- 5 116 326
- US-A- 5 492 536
- US-A- 5 562 626

## Description

La présente invention concerne un ensemble de sécurité pour une seringue pré-remplie d'injection de liquide, notamment d'un médicament.

On connaît déjà dans l'état de la technique un ensemble de sécurité pour une seringue d'injection de liquide pré-remplie comportant un corps tubulaire, formant un réservoir pour le liquide, portant une aiguille d'injection du liquide, et un poussoir monté déplaçable dans le corps entre des positions d'attente et de fin d'injection.

Habituellement, le poussoir de la seringue comporte une extrémité de manoeuvre externe au corps de la seringue et une extrémité interne au corps de cette seringue portant un piston. L'extrémité de manoeuvre du poussoir est encore appelée tête de poussoir.

Certains produits médicaux tels que les vaccins, les héparines et leurs dérivés et autres médicaments à dosages précis, ne peuvent être stockés dans les containeurs ou seringues en plastique par suite de taux de reprise d'humidité qui risquent d'en compromettre la composition.

Conscients de ces impératifs, les laboratoires sont astreints à n'utiliser que des containeurs en verre, qui ne présentent aucune porosité. Compte tenu des maladies infectieuses à virus jusqu'alors sans antidote, une sécurité est actuellement recherchée, permettant une protection de tous les personnels soignants contre les risques de contaminations éventuelles lors d'accidents professionnels dus à des piqûres. Cette protection doit être proche du taux de 100% réclamé par la profession.

Le problème est donc d'adapter un mécanisme de sécurité sur des seringues de préférence en verre, notamment de 0,1 à 2 ml de capacité, tout type de mécanisme interne à ces seringues étant exclu.

D'autre part, l'impératif de stérilité interdit toute manipulation interne de la seringue après remplissage.

Quelques solutions ont été présentées aux laboratoires et écartées pour différents motifs : augmentation du volume, modification des lignes de remplissage, opérations complexes pour le médecin ou les personnels devant mettre ces produits en oeuvre, pseudo-protection plus nuisible qu'utile.

On notera les libertés de langage criticables prises par de nombreux inventeurs dans les appellations telles que : « seringues à aiguille auto-rétractable », qui en fait ne sont, très souvent, que des seringues auto-blocantes, ou à éléments destructibles, ou à capuchon ou tube coulissant occultant l'aiguille après usage.

Dans la pratique une infirmière tient une seringue entre l'index et le majeur et appuie avec le pouce sur la tête du poussoir. De ce fait, la seringue étant maintenue, la rétraction de l'aiguille ne peut s'opérer que dans un poussoir ou piston creux, le corps de la seringue demeurant immobile.

Dans les autres cas, un fourreau coulissant longitudinalement sur l'extérieur de la seringue vient « coiffer » l'aiguille ce qui présente en fait un « escamotage de l'aiguille » par avancement du fourreau soit manuel, soit automatique.

Cet agencement connu est à la base de nombreux brevets, et, a été réfuté par la profession médicale par suite du risque d'arrachage ou d'incision résultant du choc du fourreau venant en biais percuter le patient sous la force d'un ressort de rappel du fourreau en position d'escamotage de l'aiguille.

US-A-5562 626 décrit un ensemble de sécurité du type défini dans le preambule de la revendication 1.

L'invention a pour but de remédier aux inconvénients exposés ci-dessus, tout en répondant aux critères suivants : faible encombrement, adaptation en fin de chaîne avant emballage, sécurité à déclenchement unimanuel, automatique, à commande indépendante de la volonté humaine, protection la plus voisine des 100% souhaités.

A cet effet, l'invention a pour objet un ensemble de sécurité, du type décrit dans US-A-5 562 626, comprenant les caractéristiques définies dans la partie caractérisante de la revendication 1.

Suivant d'autres caractéristiques de cet ensemble :
- le fourreau porte des moyens de préhension externes destinés à être saisis par les doigts d'un utilisateur pour injecter le liquide par rapprochement axial d'une extrémité de manoeuvre du poussoir et des moyens de préhension ;
- les moyens de préhension du fourneau comprennent deux ailettes ou oreilles sensiblement diamétralement opposées ;
- les moyens de préhension comprennent un épaulement ménagé sur la surface externe du fourreau ;
- les moyens de rappel du corps de la seringue comprennent un ressort de poussée destiné à venir en appui, d'une part, sur un épaulement interne d'appui ménagé dans le fourreau entre le siège d'appui de la collerette et l'extrémité distale de ce fourreau, et d'autre part, sur la collerette du corps de seringue ;
- le capuchon comprend des moyens d'assujettissement au poussoir ;
- le capuchon et l'extrémité proximale du fourreau comprennent des moyens complémentaires de limitation de la course du capuchon s'opposant à la force élastique des moyens de rappel du corps après libération des moyens de blocage ;
- les moyens complémentaires de limitation de la course du capuchon comprennent des épaulements annulaires complémentaires, formant butées, ménagés dans le fourreau et la capuchon ;
- les moyens complémentaires de limitation de la course du capuchon comprennent des moyens complémentaires d'encliquatage portés par le capuchon et le fourreau ;
- le fourreau et le capuchon ont des formes générales de révolution et comprennent des moyens complémentaires d'immobilisation en rotation de l'un par rapport à l'autre ;
- les moyens complémentaires d'immobilisation en rotation du fourreau et du capuchon comprennent au moins une rainure longitudinale ménagée dans le capuchon coopérant avec un doigt correspondant solidaire du fourreau ;
- les moyens complémentaires d'immobilisation en rotation du fourreau et du capuchon comprennent au moins une lumière axiale ménagée dans la capuchon, coopérant avec une ailette ;

L'invention a également pour objet un dispositif comprenant les caractéristiques définies dans la revendication 13.

L'invention sera mieux comprise à la lecture de la description qui va suivre donnée uniquement à titre d'exemple et faite en se référant aux dessins dans lesquels :
- les figures 1 à 3 sont des vues en coupe axiale d'un ensemble de sécurité pour une seringue selon un premier mode de réalisation de l'invention dans trois configurations d'utilisation respectivement ;
- les figures 4 à 6 sont des vues en coupe axiale d'un ensemble de sécurité pour une seringue selon un deuxième mode de réalisation de l'invention, dans trois configurations d'utilisation respectivement ;
- la figure 7 est une vue en coupe axiale du fourreau de protection de l'ensemble d'injection représenté sur les figures 4 à 6 ;
- les figures 8 et 9 sont des vues du capuchon de l'ensemble d'injection représenté sur les figures 4 à 6, en coupe axiale suivant deux plans perpendiculaires entre eux.

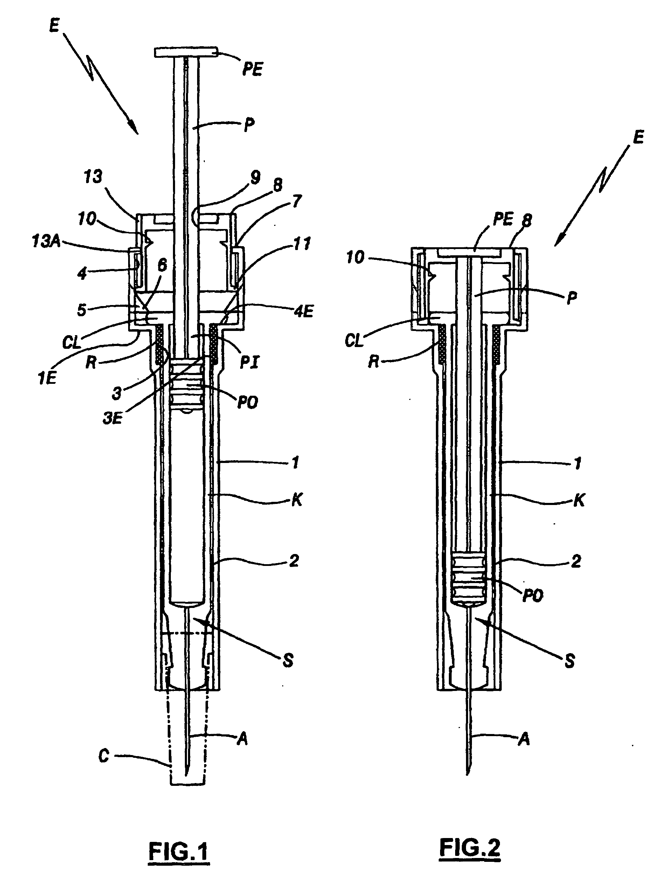

On a représenté sur les figures 1 à 3 un ensemble de sécurité pour une seringue classique s d'injection de liquide, notamment un liquide médical, selon un premier mode de réalisation de l'invention. Cet ensemble de sécurité est désigné par la référence générale E.

La seringue S est pré-remplie, prête à l'emploi, et comporte un corps tubulaire K, formant un réservoir pour le liquide, portant une aiguille A d'injection du liquide, et un poussoir P monté déplaçable dans le corps K entre une position d'attente, telle que représentée sur la figure 1, et une position de fin d'injection de liquide, telle que représentée sur la figure 2.

Le poussoir P comporte une première extrémité PE, externe au corps K, et une seconde extrémité PI, interne au corps K, portant un piston classique PO. L'extrémité externe PE forme une extrémité de manoeuvre du poussoir.

L'ensemble E comporte un fourreau de sécurité tubulaire dans lequel est logé la seringue, notamment le corps K. Le fourreau, de révolution, est constitué par exemple, par un tube cylindrique 1 en matière plastique ou en tout autre matériau ayant une transparence suffisante pour permettre la visualisation du liquide contenu dans le corps K de la seringue ainsi que d'une étiquette éventuelle d'identification du médicament. On notera que le tube cylindrique 1 est étagé.

Dans ce qui suit un élément sera qualifié de proximal ou distal selon qu'il est proche ou éloigné axialement de l'extrémité externe PE du poussoir.

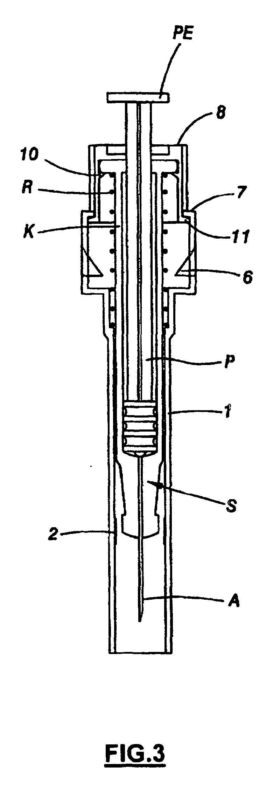

Le corps K est déplaçable axialement dans le tube 1 entre une position active dans laquelle l'aiguille A fait saillie à travers une extrémité distale (supérieure) du tube 1, et une position de protection dans laquelle l'aiguille A est escamotée dans le tube 1. La position active est représentée sur les figures 1 et 2. La position de protection est représentée sur la figure 3.

On notera que le corps K de la seringue comporte une extrémité distale portant l'aiguille A et une extrémité proximale munie d'une collerette CL.

La surface interne du tube 1 comporte trois guides 2 en relief disposés dans le sens longitudinal de ce tube 1, espacés entre eux de 120°, destinés à centrer le corps K de la seringue, réduire le jeu et améliorer le coulissement du corps K dans le tube 1, tout en permettant le passage d'une cape en caoutchouc classique C (représentée en traits mixtes sur la figure 1) protégeant l'aiguille A avant utilisation de la seringue.

L'extrémité proximale du tube 1 porte, de bas en haut, un premier logement interne de révolution 3 prolongé par un second logement interne de révolution 4 de diamètre supérieur à celui du premier logement interne.

Le corps K est immobilisé par rapport au tube 1 à l'aide de moyens de blocage comprenant, d'une part, un siège 4E d'appui de la collerette CL formé par un épaulement interne délimitant le second logement 4, et d'autre part, des moyens escamotables de serrage de la collerette CL contre ce siège 4E, portés par l'extrémité proximale du tube 1.

Dans l'exemple illustré sur les Figures 1 à 3, les moyens de serrage escamotables sont formés par deux pattes 5, sensiblement diamétralement opposées, partiellement découpées dans la paroi du tube 1 délimitant le second logement 4. Les pattes 5 sont terminées par des ergots escamotables 6 destinés à maintenir la collerette CL du corps de la seringue en appui contre le siège 4E. On notera que les pattes 5 sont pliées de façon à s'étendre sensiblement dans un plan longitudinal diamétral du tube. En variante, les moyens de serrage escamotables pourraient ne comporter qu'une seule patte 5.

Le corps K de la seringue est rappelé élastiquement vers sa position de protection par un ressort de poussée R disposé dans le premier logement 3. Ce ressort R est en appui, d'une part, sur un épaulement interne 3E, délimitant le premier logement interne 3, et d'autre part, sur la collerette CL du corps K de la seringue. L'épaulement interne d'appui 3E est disposé entre le siège 4E d'appui de la collerette CL et l'extrémité distale du tube 1.

Sur la Figure 1, qui représente l'ensemble de sécurité et la seringue prêts à être utilisés, le ressort R est comprimé. Ce ressort reste dans cet état jusqu'à la fin de l'injection du liquide.

Les ergots 6 permettent donc d'immobiliser le corps K par rapport au tube 1 en position active en s'opposant à la force élastique de rappel du ressort R.

On notera que l'extrémité proximale du second logement interne 4 est délimitée par un anneau intérieur 7 venu de moulage avec le tube 1.

Les moyens de blocage du corps K de la seringue par rapport au tube 1 sont libérables à l'aide de moyens activés lorsque le poussoir P est en position de fin d'injection.

Dans l'exemple illustré sur les figures 1 à 3, les moyens de libération des moyens de blocage comprennent un capuchon de révolution 8, de préférence en matière plastique, monté coulissant axialement dans le second logement 4 du tube 1, entre une position haute d'attente, telle que représentée sur la figure 1, et une position d'escamotage des ergots 6, telle que représentée sur la figure 2. Le capuchon 8 est traversé par le poussoir P qui s'étend à travers un trou 9 ménagé dans l'extrémité proximale de ce capuchon 8.

Le capuchon 8 comporte également des ergots intérieurs 10 espacés entre eux de 90°, ainsi qu'un anneau extérieur 11 venu de moulage avec l'extrémité distale de ce capuchon.

L'anneau intérieur 7 du second logement 4 et l'anneau extérieur 11 du capuchon 8 délimitent des épaulements annulaires complémentaires, formant butées, limitant la course de ce capuchon 8, après libération des moyens de blocage du corps K, en s'opposant à la force élastique de rappel du ressort R (voir figure 3). Les anneaux complémentaires 7, 11 font donc office de limiteur de retour du capuchon 8.

De préférence, selon le type de seringue notamment, le tube 1 et le capuchon 8 comportent des moyens complémentaires d'immobilisation en rotation de l'un par rapport à l'autre. Ces moyens complémentaires d'immobilisation en rotation comprennent, par exemple, au moins une rainure longitudinale 13, ménagée dans le capuchon 8, coopérant par emboîtement avec un doigt correspondant 13A prolongeant radialement l'anneau intérieur 7 du second logement 4. De préférence, le capuchon comporte deux rainures 13 diamétralement opposées comme cela est illustré sur les figures 1 à 3.

La mise en place de la seringue dans l'ensemble de sécurité E peut être réalisée de la façon suivante.

Initialement, le corps K de la seringue est rempli de liquide à injecter. Ce liquide est retenu dans le corps K par le piston PO. Le poussoir P est séparé de ce piston.

On place le corps K de la seringue pré-remplie dans le tube 1 en encliquetant la collerette CL sous les ergots 6 après avoir comprimé le ressort R dans le premier logement 3.

Ensuite, le capuchon 8 est engagé dans le second logement 4 en étant par exemple enfoncé dans ce second logement 4 jusqu'à ce que l'anneau extérieur 11 arrive au contact des ergots 6, comme cela est représenté sur la figure 1.

Enfin, le poussoir P de la seringue est relié au piston PO en introduisant l'extrémité distale de ce poussoir dans le capuchon 8 et le tube 1, à travers l'orifice 9 de ce capuchon 8.

L'ensemble de sécurité E est alors prêt à l'emploi dans la configuration illustrée sur la figure 1, l'aiguille A de la seringue faisant saillie à travers l'extrémité distale du tube 1.

Afin d'obtenir un effet d'aiguille et de seringue auto-rétractable, il est impératif que la préhension de l'ensemble de sécurité E soit réalisée au niveau du tube de protection 1 ce qui permet à la seringue munie de son aiguille de se rétracter librement, parfaitement en ligne, et dès la fin de l'injection, c'est-à-dire sans risque pour le patient. C'est ainsi que l'on obtient un ensemble E correctement désigné comme étant du type à aiguille auto-rétractable.

La préhension au niveau du tube 1 est réalisée à l'aide de moyens de préhension externes, portés par le tube 1, destinés à être saisis par les doigts d'un utilisateur pour injecter le liquide par rapprochement axial de l'extrémité externe PE du poussoir et des moyens de préhension.

Dans l'exemple illustré sur les figures 1 à 3, les moyens de préhension du tube 1 comprennent un épaulement 1E ménagé sur la surface externe du tube 1.

Pour réaliser l'injection du liquide, l'utilisateur saisit l'ensemble E entre l'index et le majeur. Le pouce exerce une pression de la seringue sur l'extrémité externe PE du poussoir. L'index et le majeur retiennent le tube 1 en saisissant l'épaulement de préhension 1E.

L'utilisateur enfonce le poussoir PE dans le corps de la seringue en rapprochant axialement l'extrémité PE du poussoir de l'épaulement de préhension 1E, ceci jusqu'à atteindre la position de fin d'injection représentée sur la figure 2.

La course de déplacement du poussoir P depuis sa position d'attente jusqu'à sa position de fin d'injection comporte une course finale d'assujettissement de ce poussoir P au capuchon 8 précédée d'une course morte initiale du poussoir P par rapport au capuchon 8 au cours de laquelle ce capuchon 8 reste immobile. Au cours da la course d'assujettissement du poussoir P au capuchon 8, l'extrémité externe PE du poussoir, formant tête d'assujettissement, coopère avec une lamage délimitant le bord de l'orifice 9 du capuchon.

Du fait de l'entraînement du capuchon 8 par le poussoir P, l'anneau extérieur 11 de ce capuchon coopère avec les ergots 6 de façon à les escamoter, comme cela est représenté sur la figure 2. Sur cette figure 2, on voit que le corps K de la seringue est vidé de son contenu, le liquide ayant été injecté dans le corps d'un patient par l'utilisateur. Le poussoir P est en fin de course.

Du fait de l'escamotage des ergots 6, et du relâchement de la pression exercée par le pouce de l'utilisateur sur l'extrémité externe PE du poussoir, le ressort R se détend et pousse la seringue vide vers le haut (en considérant les figures) de façon à encliqueter la collerette CL du corps entre le bord de l'orifice E du capuchon et les ergots 10 comme cela est représenté sur la figure 3. Le capuchon 8 se déplace également sous l'effet de la force de rappel du ressort R vers la position représentée sur la figure 3 dans laquelle les anneaux 7, 11 coopèrent entre eux pour limiter la course du capuchon 8 et donc la course de rétraction de la seringue. Dans la configuration illustrée sur la Figure 3, l'aiguille A est escamotée dans le tube 1 en réalisant ainsi une sécurité à 100%.

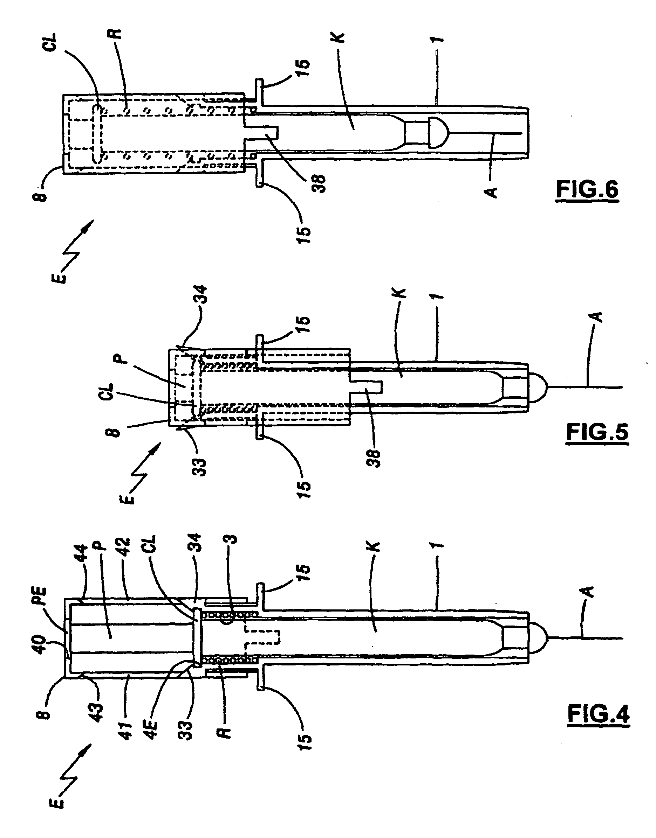

Sur les figures 4 à 9, on a représenté un ensemble de sécurité E selon un deuxième mode de réalisation de l'invention. Ce dernier mode de réalisation permet notamment l'utilisation d'une seringue comportant un vaccin.

Sur les figures 4 à 9 les éléments analogues à ceux des figures précédentes, sont désignés par des références identiques.

Sur les figures 4 à 6, on reconnaît les éléments analogues à ceux du mode de réalisation précédent, à savoir le tube 1 comportant le logement 3 dans lequel est disposé le ressort R. L'extrémité proximale du tube. 1 est prolongée par deux ergots 33, 34, de préférence diamétralement opposés. En variante, le nombre d'ergots 33, 34 peut être inférieur ou supérieur à deux, Les ergots 33, 34 constituent des moyens de blocage ayant une fonction d'immobilisation du corpo K de la seringue par rapport au tube par serrage de la collerette CL de ce corps entre les ergots 33, 34 et le siège d'appui 4E. La fonction des ergots 33, 34 est donc analogue à celles des ergots 6 du premier mode de réalisation de l'invention.

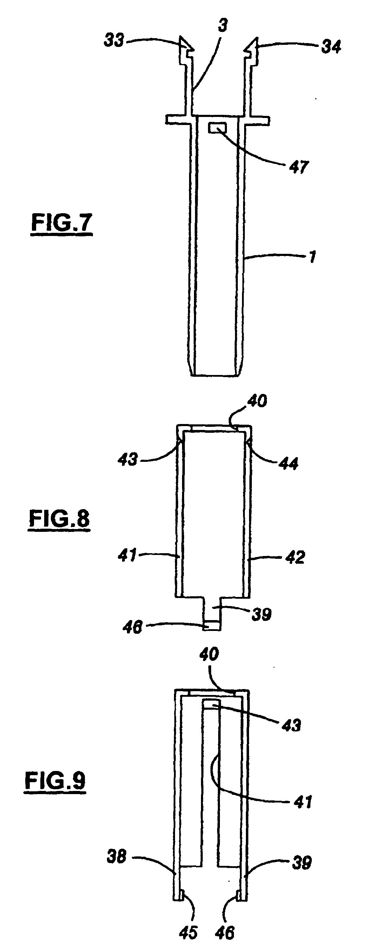

Deux ailettes de réhension 15 sont venues de moulage avec l'extrémité proximale du tube 1 et disposées à 180° l'une de l'autre. Le tube 1 est représenté isolé de l'ensemble E sur la figure 11. Le capuchon 8 (voir notamment figures 8 et 9) comporte dans ce cas deux languettes longitudinales 38, 39, sensiblement diamétralement opposés, prolongeant son extrémité distale. L'extrémité proximale du capuchon 8 est percée d'un trou 40. Le capuchon 8 comporte en outre deux lumières longitudinales verticales 41, 42 décalées de 90° par rapport aux languettes 38, 39.

Les lumières 41, 42 coopèrent par emboîtement avec les ailettes 15 de manière à former des moyens complémentaires d'immobilisation en rotation du capuchon 8 par rapport au tube 1.

Les ergots 33, 34 sont escamotables, de façon à libérer la collerette CL, par coopération avec des rampes 43, 44 limitant les extrémités proximales des lumières 41, 42.

Le trou 40 du capuchon permet le montage du poussoir dans l'ensemble d'injection. Le poussoir P est assujetti au capuchon 8 à l'aide de moyens classiques non représentés.

La figure 4 représente l'ensemble de sécurité E dans une configuration prête à son utilisation, l'aiguille de la seringue faisant saillie à travers l'extrémité distale du tube 1. Partant de cette configuration, l'utilisateur réalise une injection, notamment en intra-musculaire ou en sous-cutanée, en tenant le tube 1 entre l'index et le majeur, grâce aux ailettes 15, tout en appuyant avec le pouce sur l'extrémité externe PE du poussoir. Le capuchon 8 étant assujetti au poussoir P, ce capuchon s'enfonce sous l'effet de la pression du pouce dans le même sens que le poussoir P jusqu'à atteindre la position représentée sur la figure 5.

Lorsque le poussoir P atteint sa position de fin d'injection, telle que représentée sur la figure 5, le capuchon 8 écarte les ergots 33, 34 par coopération des rampes 43, 44 du capuchon 8 avec des ergots 33, 34, ce qui a pour effet de libérer le ressort R.

Le ressort pousse alors le corps de la seringue vide vers l'extrémité proximale du capuchon 8, comme cela est représenté sur la figure 6.

Après libération des ergots 33, 34, la course du capuchon 8 est limitée par des moyens d'encliquetage o'opposant à la force élastique de rappel du ressort R. Ces moyens d'encliquetage comprennent des plots de blocage 45, 46 ménagés sur les extrémités libres des languettes 38, 39 coopérant par emboîtement avec des trous 47, diamétralement opposés ménagés sur l'extrémité proximale du tube 1 (voir figure 7).

Sur la figure 6 qui illustre la configuration de l'ensemble de sécurité E après utilisation, on voit que l'aiguille est escamotée dans le tube 1.

Dans le deuxième mode de réalisation de l'invention, la seringue et l'aiguille sont bien auto-rétractables, le tube 1 constituant une partie stable servant d'appui fixe.

On notera que le tube 1 par ses ailettes 33., 36 assure une stabilité sur une surface non plane.

.Les modes de réalisation décrits ci-dessus assurent la rétraction de l'aiguille parfaitement en ligne par rapport à son introduction, tout en remplissant une fonction d'anti-accident pour les personnels soignants.

Toute contagion accidentelle devient impossible, l'extrémité distale du tube 1 ayant un diamètre réduit, mesurant par exemple 9 mm, ce qui interdit l'introduction accidentelle d'un doigt d'un utilisateur. La sécurité de l'ensemble E atteint donc pratiquement 100%, et, conformément au but de l'invention, le déclenchement de la sécurité (escamotage de l'aiguille) est automatique et indépendant de la volonté humaine.

D'autre part, l'escamotage de l'aiguille est réalisé d'une seule main, sans geste spécial, en respectant les règles médicales ainsi que les pratiques des personnels soignants.

L'invention permet aussi bien aux soignants qu'aux personnes devant manipuler, convoyer ou détruire les seringues usagées, d'éviter tout risque d'accidents.

## Revendications

1. Ensemble de sécurité pour une seringue d'injection de liquida pré-remplie (S) comportant un corps tubulaire(K), formant un réservoir pour le liquide, portant une aiguille (A) d'injection du liquide, et un poussoir (P) monté déplaçable dans le corps (K) entre des positions d'attente et de fin d'inaction, le corps (K) de la seringue comportant une extrémité proximale munie d'une collerette (CL), l'ensemble comprenant
un fourreau tubulaire (1) dans lequel le corps (K) de la seringue est destiné à être logé déplaçable axialement entre une position active, dans laquelle l'aiguille (A) fait saillie à travers une extrémité distale du fourreau (1), et une position de protection, dans laquelle l'aiguille (A) est escamotée dans le fourreau (1),
des moyens de rappel élastique (R) du corps (K) vers sa position de protection,
et des. moyens de blocage (6 ; 33, 34) destinés à immobiliser le corps (K) par rapport au fourreau (1) en position active en s'opposant à la force élastique des moyens de rappel, ces moyens de blocage étant libérés par des moyens (8) de libération lorsque le poussoir (P) est en position de fin d'injection,
**caractérise en ce que** les moyens de blocage comprennent un siège (4E) destiné à faire un appui pour la collerette (CL) ménagé dans une extrémité proximale du fourreau (1) et des moyens escamotables (6 ; 33, 34) de serrage de la collerette (CL) contre le siège (4E), portés par cette extrémité proximale du fourreau (1),
les moyens de serrage escamotables comprenant au moins un ergot escamotable (6 ; 33, 34),
les moyens de libération des moyens de blocage comprenant un capuchon (8) monté coulissant axialement sur l'extrémité proximale du fourreau (1) entre une position d'attente et une position d'escamotage de l'ergot de serrage (6 ; 33, 34), le capuchon (8) étant destiné à être assujetti au poussoir (P) pendant au moins une partie de la course de déplacement de ce poussoir (P) depuis sa position d'attente jusqu'à sa position de fin d'injection.

2. Ensemble selon la revendication 1, **caractérisé en ce que** le fourreau (1) porte des moyens de préhension externes (1E ; 15) destinés à être saisis par les doigts d'un utilisateur pour injecter le liquide par rapprochement axial d'une extrémité de manoeuvre (PE) du poussoir et des moyens de préhension (1E; 15).

3. Ensemble selon la revendication 2, **caractérisé en ce que** les moyens de préhension du fourreau (1) comprennent deux ailettes ou oreilles (15) sensiblement diamétralement opposées.

4. Ensemble selon la revendication 2, **caractérisé en ce que** les moyens de préhension comprennent un épaulement (1E) ménagé sur la surface externe du fourreau (1).

5. Ensemble selon l'une quelconque des revendications précédentes, **caractérisé en ce que** les moyens de rappel du corps (K) de la seringue en position de protection comprennent un ressort de poussée (R) destiné à venir en appui, d'une part, sur un épaulement interne d'appui (3E) ménagé dans le fourreau (1) entre le siège (4E) d'appui de la collerette et l'extrémité distale de ce fourreau (1), et d'autre part, sur la collerette (CL) du corps (K) de seringue.

6. Ensemble selon l'une quelconque des revendications précédentes, **caractérisé en ce que** le capuchon (8) comprend des moyens d'assujettissement au poussoir (P).

7. Ensemble selon l'une quelconque des revendications précédentes, **caractérisé en ce que** le capuchon (8) et l'extrémité proximale du fourreau comprennent des moyens complémentaires (7, 11 ; 38, 39, 47) de limitation de la course du capuchon (8) s'opposant à la force élastique des moyens de rappel du corps (K) après libération des moyens de blocage (6 ; 33, 34).

8. Ensemble selon la revendication 7, **caractérisé en ce que** les moyens complémentaires de limitation de la course du capuchon comprennent des épaulements annulaires complémentaires (7, 11), formant butées, ménagés dans le fourreau (1) et le capuchon (8).

9. Ensemble selon la revendication 7, **caractérisé en ce que** les moyens complémentaires de limitation de la course du capuchon comprennent des moyens complémentaires d'encliquetage (38, 39, 47) portés par le capuchon (8) et le fourreau (1).

10. Ensemble selon l'une quelconque des revendications précédentes, **caractérisé en ce que** le fourreau (1) et le capuchon (8) ont des formes générales de révolution et comprennent des moyens complémentaires (13, 13A ; 15, 41, 42) d'immobilisation en rotation de l'un par rapport à l'autre.

11. Ensemble selon la revendication 10, **caractérisé en ce que** les moyens complémentaires d'immobilisation en rotation du fourreau et du capuchon comprennent au moins une rainure longitudinale (13) ménagée dans le capuchon (8) coopérant avec un doigt correspondant (13A) solidaire du fourreau (1).

12. Ensemble selon les revendications 3 et 10 prises ensemble, **caractérisé en ce que** les moyens complémentaires d'immobilisation en rotation du fourreau et du capuchon comprennent au moins une lumière axiale (41, 42) ménagée dans le capuchon (8), coopérant avec une ailette (15).

13. Dispositif d'injection comprenant une seringue d'injection de liquide pré-remplie (S) comportant un corps tubulaire(K), formant un réservoir pour le liquide, portant une aiguille (A) d'injection du liquide, et un poussoir (P) monté déplaçable dans le corps (K) entre des positions d'attente et de fin d'injection, et un ensemble de sécurité suivant l'une quelconque des revendications 1 à 12.

## Claims

1. A safety assembly for a prefilled syringe (S) for injecting liquid, the syringe comprising a tubular body (K) forming a reservoir for the liquid, carrying a needle (A) for injecting the liquid, and having a plunger (P) mounted in the body (K) to be movable between a ready position and and end-of-injection position, the body (K) of the syringe having a proximal end provided with a flange (CL), the assembly comprising :
a tubular sheath (1) in which the body (K) of the syringe is designed to be housed in axially displaceable manner between an active position in which the needle (A) projects through a distal end of the sheath (1) and a protection position in which the needle (A) is retracted inside the sheath (1) ;
resilient return means(R) for urging the body (K) towards its protection position ; and
locking means (6 ; 33, 34) for preventing the body (K) from moving relative to the sheath (1) in the active position by opposing the resilient force of the return means, said locking means being released by release means (8) when the plunger (P) is in its end-of-injection position,
**characterized in that** the locking means comprise a seat (4E) designed to make support for the flange (CL), which seat is formed in a proximal end of the sheath (1), and also comprising retractable means (6 ; 33, 34) for clamping the flange (CL) against the seat (4E), said means being carried by said proximal end of the sheath (1) ; and
the retractable clamping means comprising at least one retractable catch (6 ; 33, 34),
the means for releasing the locking means comprising a cap (8) mounted to slide axially on the proximal end of the sheath (1) between a ready position and a position for retracting the clamping catch (6 ; 33, 34), the cap (8) being designed to be secured to the plunger (P) at least during a portion of the displacement stroke of the plunger (P) from its ready position to its end-of-injection position.

2. An assembly according to claim 1, **characterized in that** the sheath (1) carries external retention means (1E ; 15) for being held by the fingers of a user to inject the liquid by moving a drive end (PE) of the plunger axially towards the retention means (1E ; 15).

3. An assembly according to claim 2, **characterized in that** the retention means of the sheath (1) comprise two substantially diametrically opposite fins or lugs (15).

4. An assembly according to claim 2, **characterized in that** the retention means comprise a shoulder (1E) formed on the outside surface of the sheath (1).

5. An assembly according to any preceding claim, **characterized in that** the means for resiliently urging the body (K) of the syringe into the protection position comprise a thrust spring (R) designed to bear both against the flange (CL) of the body (K) of the syringe and also against an internal bearing shoulder (3E) formed in the sheath (1) between the bearing shoulder (4E) for the flange and the distal end of the sheath (1).

6. An assembly according to any preceding claim, **characterized in that** the cap (8) has means for securing it to the plunger (P).

7. An assembly according to any preceding claim, **characterized in that** the cap (8) and the proximal end of the sheath comprise complementary means (7, 11 ; 38,39,47) for limiting the stroke of the cap (8) in opposition to the resilient force of the means for returning the body (K) after the locking means (6 ; 33, 34) have been released.

8. An assembly according to claim 7, **characterized in that** the complementary means for limiting the stroke of the cap comprise complementary annular shoulders (7, 11) forming abutments that are provided on the sheath (1) and the cap (8).

9. An assembly according to claim 7, **characterized in that** the complementary means for limiting the stroke of the cap comprise complementary snap-fastening means (38, 39, 47) carried by the cap (8) and the sheath (1).

10. An assembly according to any preceding claim, **characterized in that** the sheath (1) and the cap (8) are generally in the form of bodies of revolution and have complementary means (13, 13A ; 15, 41, 42) for preventing relative rotation between each other.

11. An assembly according to claim 10, **characterized in that** the complementary means for preventing relative rotation of the sheath and the cap comprise at least one longitudinal groove (13) formed in the cap (8) and co-operating with a corresponding finger (31A) secured to the sheath (1).

12. An assembly according to claimS 3 and 10 taken together, **characterized in that** the complementary means for preventing relative rotation of the sheath and the cap comprise at least one axial slot (41, 42) formed in the cap (8) and co-operating with a fin (15).

13. An injection device comprising a prefilled syringe (S) for injecting liquid, the syringe comprising a tubular body (K) forming a reservoir for the liquid, carrying a needle (A) for injecting the liquid, and having a plunger (P) mounted in the body (K) to be movable between a ready position and an end-of-injection position, and a safety assembly according to any one of claims 1 to 12.

## Patentansprüche

1. Sicherheitseinheit für eine Spritze zum Einspritzen von vorab eingefüllter Flüssigkeit (S), die einen röhrenförmigen Körper (K) umfasst, der einen Behälter für die Flüssigkeit bildet, die eine Nadel (A) zum Einspritzen der Flüssigkeit und einen Stößel (P) umfasst, der in dem Körper (K) zwischen einer Wartestellung und einer Einspritzendstellung verschiebbar installiert ist, wobei der Körper (K) der Spritze ein proximales Ende umfasst, das mit einem Kragen (CL) versehen ist, wobei die Einheit Folgendes umfasst:
- einen röhrenförmigen Mantel (1), in dem der Körper (K) der Spritze dazu bestimmt ist, zwischen einer aktiven Position, in der die Nadel (A) durch ein distales Ende des Mantels (1) heraussteht, und einer Schutzstellung, in der die Nadel (A) in den Mantel (1) eingezogen ist, axial verschiebbar untergebracht zu sein,
- Mittel zum elastischen Zurückholen (R) des Körpers (K) zu seiner Schutzstellung,
- und Mittel zum Blockieren (6; 33, 34), die dazu bestimmt sind, den Körper (K) zu dem Mantel (1) in aktiver Position stillzustellen, indem sie sich der elastischen Kraft der Rückholmittel widersetzen, wobei diese Mittel zum Blockieren durch Mittel (B) zum Befreien freigegeben werden, wenn der Stößel (P) in Einspritzendstellung ist,
**dadurch gekennzeichnet, dass** die Mittel zum Blockieren einen Sitz (4E) umfassen, der dazu bestimmt ist, auf dem Kragen (CL), der in einem proximalen Ende des Mantels (1) eingerichtet ist, eine Auflage zu bilden, und einziehbare Mittel (6, 33, 34) zum Spannen des Kragens (CL) gegen den Sitz (4E), die von diesem proximalen Ende des Mantels (1) getragen werden,
wobei die einziehbaren Spannmittel mindestens einen einziehbaren Dorn (6, 33, 34) umfassen,
wobei die Mittel zum Befreien der Blockiermittel eine Kappe (8) umfassen, die axial auf dem proximalen Ende des Mantels (1) zwischen einer Wartestellung und einer Einziehstellung des Spanndorns (6; 33, 34) gleitend installiert ist, wobei die Kappe (8) dazu bestimmt ist, mit dem Stößel (P) während mindestens eines Teils des Verschiebungshubs dieses Stößels (P) von seiner Wartestellung bis zu seiner Endeinspritzstellung verbunden zu sein.

2. Einheit nach Anspruch 1, **dadurch gekennzeichnet, dass** der Mantel (1) äußere Mittel zum Ergreifen (1E; 15) umfasst, die dazu bestimmt sind, von den Fingern eines Benutzers ergriffen zu werden, um die Flüssigkeit durch axiales Annähern eines Manövrierendes (PE) des Stößels und der Mittel zum Ergreifen (1E; 15) einzuspritzen.

3. Einheit nach Anspruch 2, **dadurch gekennzeichnet, dass** die Mittel zum Ergreifen des Mantels (1) zwei Flügel oder Ohren (15) umfassen, die einander im Wesentlichen diametral gegenüber liegen.

4. Einheit nach Anspruch 2, **dadurch gekennzeichnet, dass** die Mittel zum Ergreifen einen Ansatz (1E) umfassen, der auf der äußeren Fläche des Mantels (1) eingerichtet ist.

5. Einheit nach einem der vorhergehenden Ansprüche, **dadurch gekennzeichnet, dass** die Rückholmittel des Körpers (K) der Spritze in Schutzposition eine Schubfeder (R) umfassen, die dazu bestimmt ist, einerseits auf einem inneren Auflageansatz (3E), der in dem Mantel (1) zwischen dem Sitz (4E) zum Aufliegen des Kragens und dem distalen Ende dieses Mantels (1) bestimmt ist, und andererseits auf dem Kragen (CL) des Körpers (K) der Spritze zum Aufliegen zu kommen.

6. Einheit nach einem der vorhergehenden Ansprüche, **dadurch gekennzeichnet, dass** die Kappe (8) Mittel zum Verbinden mit dem Stößel (P) umfasst.

7. Einheit nach einem der vorhergehenden Ansprüche, **dadurch gekennzeichnet, dass** die Kappe (8) und das proximale Ende des Mantels (1) ergänzende Mittel (7, 11; 38, 39, 47) zum Einschränken des Hubs der Kappe (8) umfassen, die sich der elastischen Kraft der Rückholmittel des Körpers (K) nach dem Freigeben der Blockiermittel (6; 33, 34) widersetzen.

8. Einheit nach Anspruch 7, **dadurch gekennzeichnet, dass** die ergänzenden Mittel zum Einschränken des Hubs der Kappe ringförmige ergänzende Ansätze (7, 11) umfassen, die Anschläge bilden, die in dem Mantel (1) und der Kappe (8) angeordnet sind.

9. Einheit nach Anspruch 7, **dadurch gekennzeichnet, dass** die ergänzenden Mittel zum Einschränken des Hubs der Kappe ergänzende Mittel zum Einrasten (38, 39, 47) umfassen, die von der Kappe (8) und dem Mantel (1) getragen werden.

10. Einheit nach einem der vorhergehenden Ansprüche, **dadurch gekennzeichnet, dass** der Mantel (1) und die Kappe (8) allgemeine Rotationsformen haben und ergänzende Mittel (13, 13A; 15, 41, 42) zum Stillstellen in Drehung zueinander umfassen.

11. Einheit nach Anspruch 10, **dadurch gekennzeichnet, dass** die ergänzenden Mittel zum Stillstellen in Drehung des Mantels und der Kappe mindestens eine Längsrille (13) umfassen, die in der Kappe (8) eingerichtet ist und mit einem entsprechenden Finger (13A) zusammenarbeitet, der fest mit dem Mantel (1) verbunden ist.

12. Einheit nach den Ansprüchen 3 und 10 gemeinsam genommen, **dadurch gekennzeichnet, dass** die ergänzenden Mittel zum Stillstellen in Drehung des Mantels und der Kappe mindestens ein axiales Langloch (41, 42) umfassen, das in der Kappe (8) eingerichtet ist und mit einem Flügel (15) zusammenarbeitet.

13. Vorrichtung zum Einspritzen, die eine Spritze zum Einspritzen einer vorab eingefüllten Flüssigkeit (S) enthält, die einen röhrenförmigen Körper (K), der für die Flüssigkeit einen Behälter bildet, umfasst, der eine Nadel (A) zum Einspritzen der Flüssigkeit und einen Stößel (P) umfasst, der in dem Körper (K) zwischen einer Wartestellung und einer Endeinspritzstellung verschiebbar installiert ist, und eine Sicherheitseinheit gemäß einem der Ansprüche 1 bis 12.
